# EUROPEAN PATENT SPECIFICATION

(11) **EP 3 046 551 B1**
(45) Date of publication and mention of the grant of the patent: **05.08.2020**
(21) Application number: 14784389.0
(22) Date of filing: 18.09.2014
(51) Int. Cl.: A61K 31/13, A61K 31/198, A61K 31/216, A61K 31/27, A61K 31/36, A61K 31/428, A61K 31/4745

(54) **TREATING NEURODEGENERATIVE DISEASE WITH FENOFIBRATE AND ANALOGS THEREOF**
BEHANDLUNG VON NEURODEGENERATIVER ERKRANKUNG MIT FENOFIBRATE UND ANALOGA DAVON
TRAITEMENT DE TROUBLE NEURODEGENERATIF AVEC FENOFIBRATE ET ANALOGUES DE CELUI-CI

(30) Priority: 18.09.2013 US 201361879553 P
(43) Date of publication of application: 27.07.2016
(73) Proprietor: Georgetown University, Washington, DC 20057 (US); Vanda Pharmaceuticals Inc., Washington, DC 20037 (US)
(72) Inventor: POLYMEROPOULOS, Mihael, Washington, District of Columbia 20037 (US); FEDEROFF, Howard J., Bethesda, Maryland 20817 (US); SU, Xiaomin, Oakton, Virginia 22124 (US)
(74) Representative: Appleyard Lees IP LLP
(86) International application number: PCT/US2014/056351
(87) International publication number: WO 2015/042286

(56) References cited:
- WO-A1-99/38498
- WO-A1-2013/071077
- WO-A1-2014/089449
- WO-A2-2004/000313
- WO-A2-2006/026785
- WO-A2-2009/147009
- DEEPAK KUMAR BHATEJA ET AL: "Peroxisome proliferator-activated receptor- activation attenuates 3-nitropropionic acid induced behavioral and biochemical alterations in rats: Possible neuroprotective mechanisms", EUROPEAN JOURNAL OF PHARMACOLOGY, ELSEVIER SCIENCE, NL, vol. 674, no. 1, 15 October 2011 (2011-10-15), pages 33-43, XP028126045, ISSN: 0014-2999, DOI: 10.1016/J.EJPHAR.2011.10.029 [retrieved on 2011-10-26]
- LIN JIANDIE ET AL: "Defects in adaptive energy metabolism with CNS-linked hyperactivity in PGC-1alpha null mice", CELL, CELL PRESS, US, vol. 119, no. 1, 1 October 2004 (2004-10-01), pages 121-135, XP002550778, ISSN: 0092-8674, DOI: 10.1016/J.CELL.2004.09.013
- YU AH HONG ET AL: "Fenofibrate Improves Renal Lipotoxicity through Activation of AMPK-PGC-1[alpha] in db/db Mice", PLOS ONE, vol. 9, no. 5, 6 May 2014 (2014-05-06), page e96147, XP055151211, DOI: 10.1371/journal.pone.0096147

## Description

### BACKGROUND

Neurodegenerative diseases can be sporadic or familial and increase in occurrence with aging. Thus, as the average life span increases across the population, the occurrence of neurodegenerative diseases increases. As many as one of four Americans is predicted to develop a neurodegenerative condition in their lifetimes. Generally, however, the underlying mechanisms causing the conditions are not well understood and few effective treatment options are available for preventing or treating neurodegenerative diseases.

### SUMMARY

Provided herein is a method of inducing proliferator-activated receptor gamma co-activator-1 alpha (PGC-1α) expression in a neural cell or population of neural cells. The method includes contacting the neural cell (e.g., a neuron or glial cell) or population of neural cells with an effective amount of fenofibrate or an analog thereof. The fenofibrate or analog thereof reduces one or more effects of oxidative stress and/or provides one or more anti-inflammatory effects. Additionally, the fenofibrate or analog thereof increases levels of phosphorylated AMPK, increases mitochondrial number, and/or increases cell viability.

Also provided herein is a method of treating or preventing a neurodegenerative disease in a subject, by administering to the subject an effective amount of fenofibrate or an analog thereof. The method optionally includes selecting a subject with a neurodegenerative disease of the central nervous system (e.g., with an early stage of the disease) or at risk for a neurodegenerative disease of the central nervous system. The effective amount of the fenofibrate or analog thereof induces PGC-1α expression in neural cells. The method optionally includes the step of determining that the subject has a reduced level of PGC-1α expression as compared to a control subject. Such a determination step can be performed before, after, or both before and after fenofibrate or an analog thereof is administered.

Also provided is a method of screening for an agent that promotes neuroprotection. The method includes contacting a cell with one or more agents to be screened and detecting PGC-1α level or activity in the cell. An increase in PGC-1α level or activity indicates the agent promotes neuroprotection.

The details of one or more embodiments of the invention are set forth in the accompanying drawings and the description below. Other features, objects, and advantages of the invention will be apparent from the description and drawings, and from the claims.

### BRIEF DESCRIPTION OF DRAWINGS

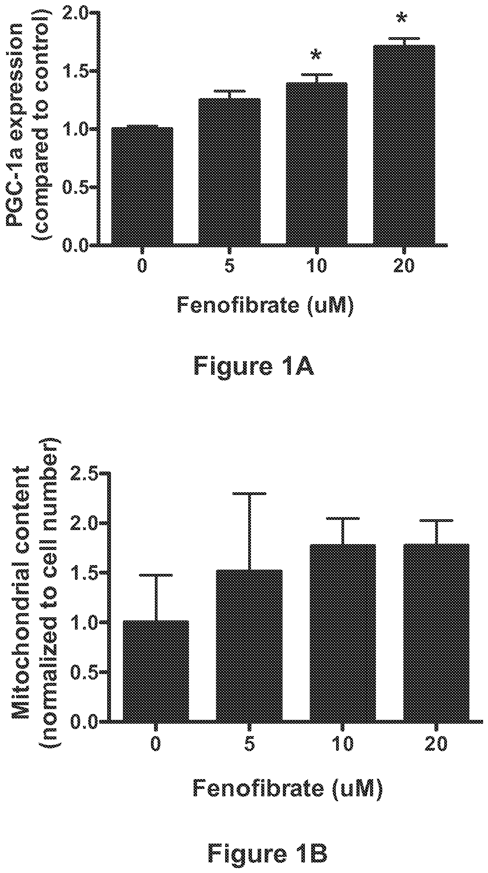

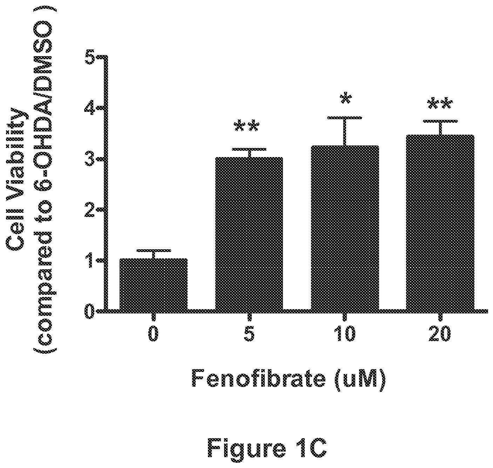

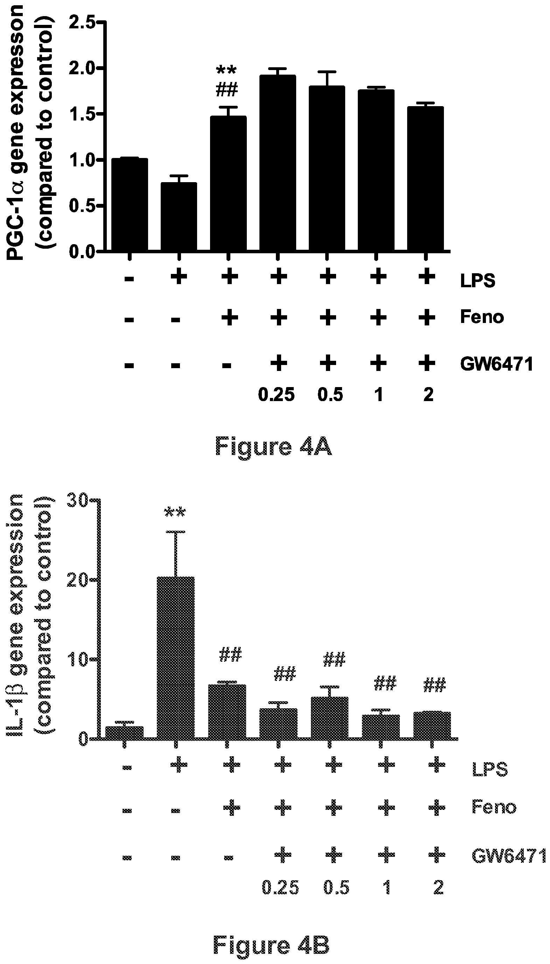

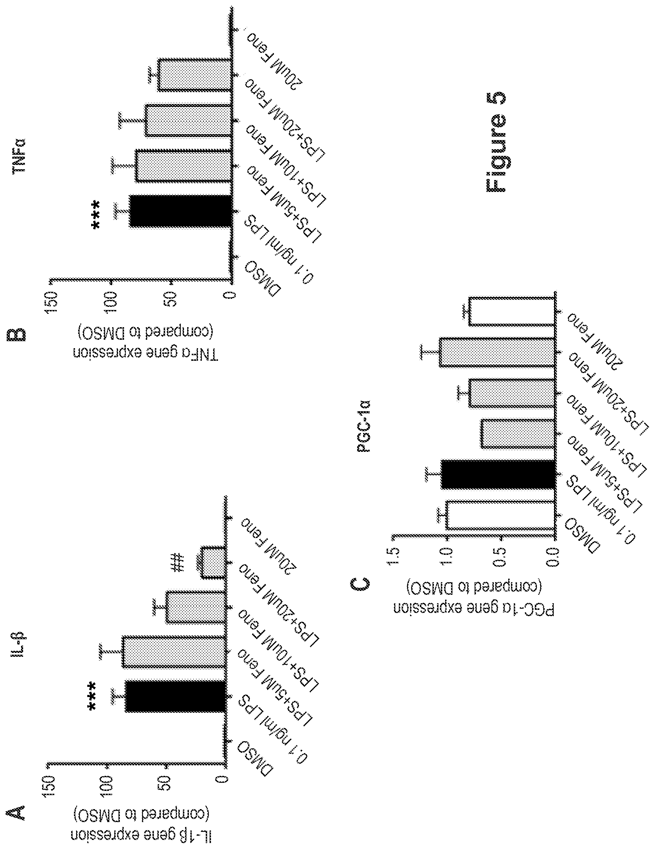

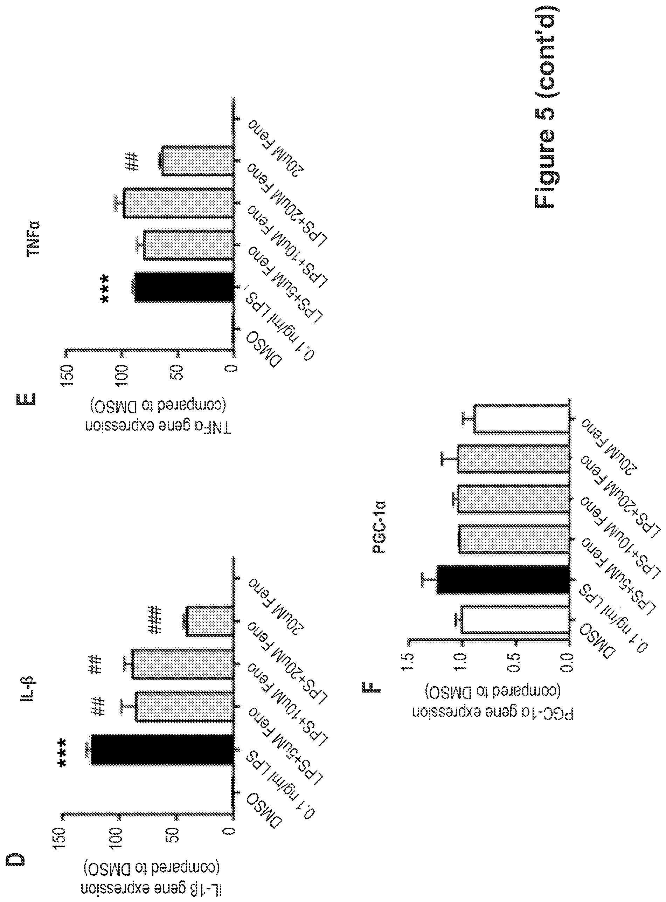

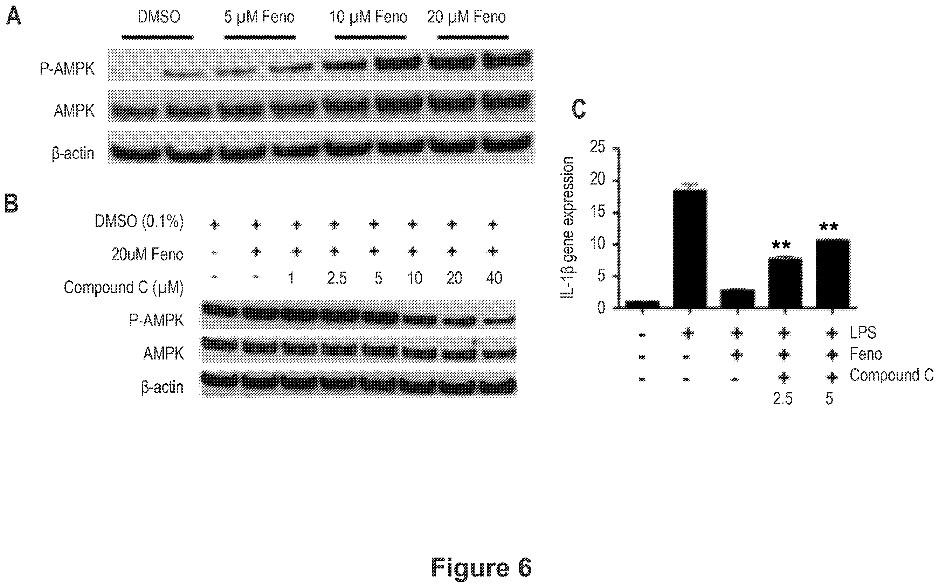

Figure 1 shows fenofibrate induces PGC-1α up-regulation and provides neuroprotection. Figure 1A shows results when MN9D cells were treated with fenofibrate for 4 hours and total RNA was extracted for PGC-1α gene expression. A significant fold increase of PGC-1α mRNA expression was detected in MN9D cells with 10 or 20 µM fenofibrate. (*, p<0.05 treatment vs no treatment control). Figure 1B shows an increase in mitochondrial contents in MN9D cells treated with fenofibrate detected by MitoTracker 96-well assay. Figure 1C shows cell viability data for MN9D cells pretreated with fenofibrate for 4 hours and then subject to 6-OHDA stress for 12 hours. Cell viability was detected by MTS assay. (* p<0.05, **p<0.01 treatment vs no treatment control).

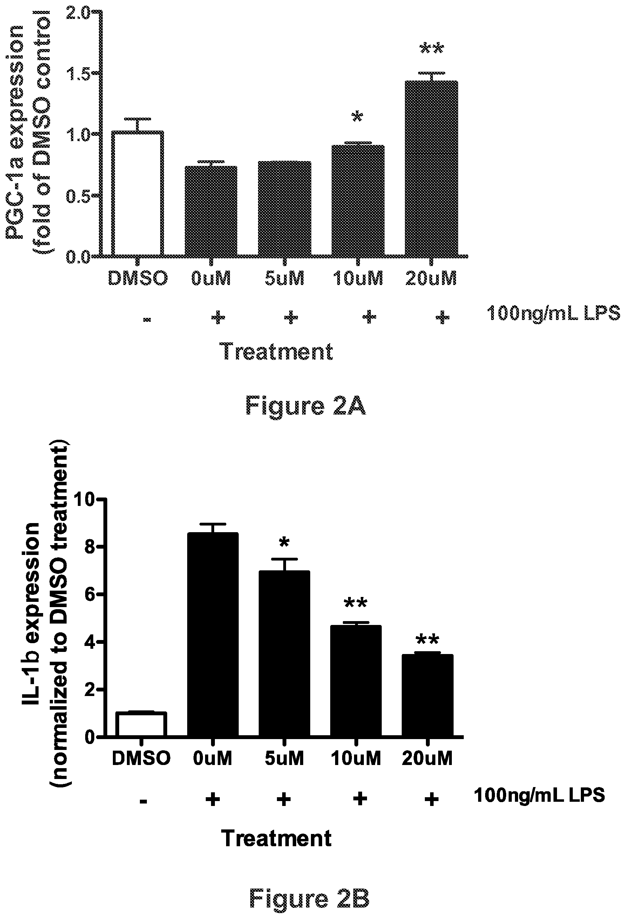

Figure 2 shows fenofibrate induces PGC-1α up-regulation and provides anti-inflammation in BV2 cells. Figure 2A shows PGC-1α expression levels in BV2 cells pretreated with fenofibrate for 4 hours and then subjected to lipopolysaccharide (LPS) for another 4 hours. PGC-1α expression was determined by RT-PCR. (*p<0.05, **p<0.01 LPS plus fenofibrate treatment vs LPS with no fenofibrate treatment). Figure 2B shows IL-1β expression in BV2 cells. (*p<0.05, **p<0.01 LPS plus fenofibrate treatment vs LPS with no fenofibrate treatment.)

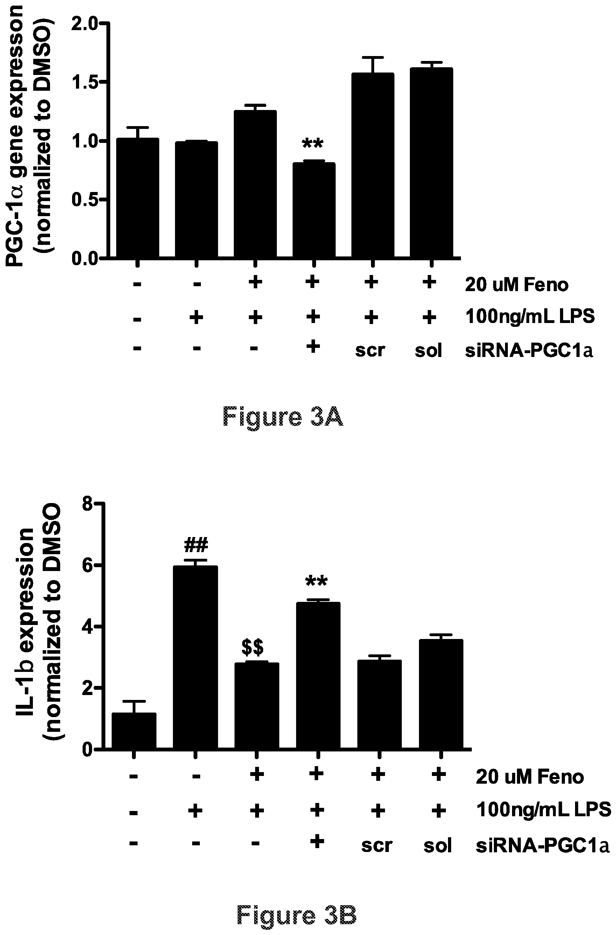

Figure 3 shows PGC-1α mediates the fenofibrate anti-inflammatory effect. BV2 cells were incubated with 20nM siRNA targeting PGC-1α for 4 hrs followed by 20 µM fenofibrate for another 18 hrs. Then the cells were treated with 100 ng/mL LPS for 4 hrs. Figure 3A shows the total RNA extracted for RT-PCR to determine the level of PGC-1α expression.
Figure 3B similarly shows the level of IL-1β mRNA expression. (##p<0.001 vs. control; $$p<0.001 vs. LPS; **p<0.001 vs. LPS+Feno. Scr, scramble, sol, RNAiMAX solution for siRNA dilution).
Figure 4 shows PPARα is not required for fenofibrate-mediated PGC-1α upregulation and anti-inflammatory effects. BV2 cells were incubated with PPARα antagonist GW6471 at various concentrations (0.25, 0.5, 1 and 2 µM) for 0.5 hrs followed by 20 µM fenofibrate for another 18 hrs. Then the cells were treated with 100 ng/mL LPS for 4 hrs. Figure 4A shows PGC-1α mRNA expression as detected from total RNA extracted for RT-PCR. Figure 4B shows and IL-1β (B) mRNA expression. (**p<0.01 vs. control; ##p<0.001 vs. LPS).
Figure 5 shows that fenofibrate inhibits LPS-induced inflammation in primary microglia derived from PGC-1α WT (PGC-1α +/+) and PGC-1α knockdown (PGC-1α +/-) mice. Figures 5A, 5B and 5C show gene expression data of primary microglia derived from PGC-1α WT (PGC-1α+/+) mice treated with fenofibrate at 5, 10, and 20 µM overnight followed by LPS for 1 hour. Figures 5D, 5E, and 5F show gene expression data for PGC-1α knockdown (PGC-1α +/-) mice were treated with fenofibrate at 5, 10, and 20 µM overnight followed by LPS for 1 hour. Total RNA was isolated and IL-1β (Figures A and D), TNFα (Figures B and E) and PGC-1α (Figures C and F) gene expression were determined by RT-PCR. (***, p<0.01, LPS vs DMSO; ##, p<0.05, ###, p<0.01, LPS+feno vs LPS, ANOVA with Student-Newman-Keuls *post hoc* analysis).
Figure 6 shows that fenofibrate enhances AMP-activated protein kinase (AMPK) phosphorylation and inhibition of AMPK weakens fenofibrate-mediated anti-inflammation effects. Figure 6A shows the levels of phosphorylated AMPK on Western blots from BV2 cells treated with various doses of fenofibrate for 1 hour. Figure 6B shows the levels of phosphorylated AMPK on Western blots from lysates of BV2 cell cultures treated with an AMPK inhibitor compound C for 0.5 hour followed by fenofibrate treatment for another 1 hour. Cell lysate was collected for Western blotting analysis. Figure 6C shows levels of IL-1β from cell cultures of BV2 cells treated with Compound C for 0.5 hour followed by fenofibrate treatment overnight. Total RNA was isolated and IL-1β gene expression was measured by RT-PCR (**, p<0.01, compared to fenofibrate and LPS, ANOVA with Student-Newman-Keuls *post hoc* analysis).

### DETAILED DESCRIPTION

Provided herein are methods of inducing PGC-1α expression in a neural cell or population of neural cells, methods of treating a subject with neurodegenerative disease.

Prior to the present invention, up-regulating PGC-1α activity was generally through gene delivery of PGC-1α overexpression. However, adenoassociated virus (AAV)-mediated overexpression of PGC-1α induced selective loss of dopaminergic markers and reduction in striatal dopamine content. Importantly, higher AAV-mediated expression of PGC-1α leads to overt degeneration of dopaminergic neurons. Super-physiological levels of PGC-1α, therefore, cause detrimental effects. Pharmacological modulation of PGC-1α induction induces PGC-1α expression or activity and can be more closely regulated. Such pharmacological modulation of PGC-1α can be titrated to moderate the increase or to normalize pathologically dysregulated PGC-1α. The present method provides a way to maintain physiological levels of PGC-1α activity by pharmacologically modulating PGC-1α expression, thereby, safely controlling PGC-1α levels by drug dosage and treatment.

The method of inducing PGC-1α expression in a neural cell or population of neural cells includes contacting the cell or population of cells with a fenofibrate or an analog thereof. The contacting step can be performed either *in vivo* or *in vitro.* Optionally, the induction of PGC-1α is independent of peroxisome proliferator-activated receptor alpha (PPAR-α) or gamma (PPAR-y) or both.

Fenofibrate or its analog can be aministered to a neural cell or populations of cells in any number of ways, including, for example, *ex vivo*, *in vitro*, and *in vivo. In vivo* administration can be directed to central or peripheral nervous system neural cells. Thus, *in vivo* contact can be useful if the subject has or is at risk of developing reduced PGC-1α levels in the central nervous system. *In vitro* contact can be desired for example in treating cells for transplantation. The neural cells can be explants from the nervous system of the same or different subject, can be derived from stem cells, or can be derived from a cell line. The neural cells can be derived from a non-neural cell that is de-differentiated and then caused to differentiate into a neural cell lineage. Such a cell can be an induced pluripotent stem cell. Because fenofibrate or its analog crosses the blood brain barrier, a neural cell in the central nervous system can be contacted with the fenofibrate or analog thereof by a systemic administration of the fenofibrate or analog to the subject. However, the fenofibrate or analog can be administered intrathecally, for example, by local injection, by a pump, or by a slow release implant.

As used herein, neural cells include both neurons (including dopaminergic neurons) and glial cells (astrocytes, oligodendrocytes, Schwann cells, and microglia). Optionally the neural cell or population of neural cells comprises central nervous system cells.

Without meaning to be limited by theory, induction of PGC-1α is associated with reduced inflammation, mitochondrial biogenesis, phosphorylation of AMPK, increased cell viability, and reduced oxidative stress. Inflammation, mitochondrial dysfunction and oxidative stress are thought to increase the likelihood a subject will develop a neurodegenerative disease (e.g., by increasing susceptibility to toxic compounds in the environment) and may hasten the progression of such a disease. Thus, the contacting step can promote neuroprotection of neural cells *in vivo* or *in vitro.* A method of treating a neurodegenerative disease as disclosed herein includes administering a fenofibrate or analog thereof to the subject. Optionally the method includes selecting a subject with a neurodegenerative disease of the central nervous. One of the advantages of the present methods is that the methods are useful even in treating a subject in the early stages of the disease. An early stage can be noted by the first signs of tremor or subtle changes in fine motor skills or muscle tone. Additional signs of Parkinson's Disease include, by way of example, olfactory dysfunction, sleep disturbances, mood change, dysautonomia, abnormal brain imaging, blood and CSF markers, and/or harbor PD-associated genetic mutation (i.e. LRRK2 and SNCA). Subjects at risk for a neurodegenerative disease include those with a family history, genetic mutation or marker, or an occupational or environmental exposure to a causative agent. Thus, those exposed for example to certain toxic compounds or to repetitive concussions would be at risk for a neurodegenerative disease.

Neurodegenerative diseases are generally marked by an insidious onset and progression of cell death and loss of function. By way of example, such diseases include without limitation Parkinson's Disease, Parkinson-plus syndrome, familial dementia, Alzheimer's Disease, Huntington's Disease, multiple sclerosis, dementia with Lewy bodies, Mild Cognitive Impairment, Pick's disease, Lewy Body disease, multiple system atrophy, progressive supranuclear palsy, amyotrophic lateral sclerosis, and retinal neurodegeneration. Parkinson-plus syndrome is selected from the group consisting of multiple system atrophy (MSA), progressive supranuclear palsy (PSP), and corticobasal degeneration (CBD).

Fenofibrate is a fibrate compound, previously used in the treatment of endogenous hyperlipidemias, hypercholesterolemias and hypertriglyceridemias. The preparation of fenofibrate is disclosed in US Patent. No. 4,058,552. Fenofibric acid is the active metabolite of fenofibrate. Fenofibrate is not soluble in water, which limits its absorption in the gastrointestinal (GI) tract. Alternative formulations and strategies have been used to overcome this problem. See U.S. Patent Nos. 4,800,079 and 4,895,726 (micronized fenofibrate); US Patent No. 6,277,405 (micronized fenofibrate in a tablet or in the form of granules inside a capsule); US Patent No. 6,074,670 (the immediate release of micronized fenofibrate in a solid state; US Patent No. 5,880,148 (combination of fenofibrate and vitamin E); US Patent No. 5,827,536 (diethylene glycol monoethyl ether (DGME) as solubilizer for fenofibrate); and US Patent No. 5,545,628 (the combination of fenofibrate with one or more polyglycolyzed glycerides. Numerous other derivatives, analogs and formulations are known to one of skill in the art. For example, other esters of p-carbonylphenoxy-isobutyric acids as described in U.S. Patent. No. 4,058,552, can be used. Fenofibrate analogs include those defined in U.S. Patent No. 4,800,079. By way of example, gemfibrozil could be used in the methods disclosed herein.

Fenofibrate is optionally dissolved in a proper solvent or solubilizers. Fenofibrate is known to be soluble in many different solubilizers, including, for example, anionic (e.g. SDS) and non-ionic (e.g. Triton X -100) surfactants, complexing agents (N-methyl pyrrolidone). Liquid and semi-solid formulations with improved bioavailability for oral administration of fenofibrate or fenofibrate derivatives are described in International Patent Application Publication No. WO2004002458.

Prolonged treatment with fenofibrate at the rate of 300 to 400 mg per day has been used but higher and lower concentration can be warranted given the condition of the subject or the level of PGC-1α that is desired. The customary adult fenofibrate dosage is three gelatin capsules per day, each containing 100 mg of fenofibrate. One of skill in the art can select a dosage or dosing regimen by selecting an effective amount of the fenofibrate or analog thereof. Such an effective amount includes an amount that induces PGC-1α expression in neural cells, an amount that has anti-inflammatory properties, an amount that reduces one or more effects of oxidative stress. Additionally, the effective amount of fenofibrate or analog thereof increases levels of phosphorylated AMPK, increases mitochondrial number, and increases cell viability.

Optionally, the fenofibrate or analog thereof is administered daily.

A method of treatment with fenofibrate or analog thereof can further comprise administering a second therapeutic agent to the subject. The second therapeutic agent is selected, for example, from the group consisting of levadopa, a dopamine agonist, an anticholinergic agent, a monoamine oxidase inhibitor, a COMT inhibitor, amantadine, rivastigmine, an NMDA antagonist, a cholinesterase inhibitor, riluzole, an anti-psychotic agent, an antidepressant, and tetrabenazine.

A method of treatment of a subject with a neurodegenerative disease optionally also includes any number of various tests before, during and/or after administration of the fenofibrate or analog thereof. For example, the subject can be tested to determine whether the subject has a reduced level of PGC-1α expression or activity as compared to a control level. A reduced level of PGC-1 can indicate that fenofibrate or an analog thereof should be administered to the client, could indicate that an increased dosage or an increased frequency of administration is needed, or could indicate that the fenofibrate or analog thereof is not sufficient treatment and an additional agent or therapeutic should be administered in combination with the fenofibrate or analog.

The method optionally includes selecting a subject with a neurodegenerative disease. One of skill in the art knows how to diagnose a subject with or at risk of developing a neurodegenerative disease. For example, one or more of the follow tests can be used: a genetic test (e.g., identification of a mutation in TDP-43 gene) or familial analysis (e.g., family history), central nervous system imaging (e.g., magnetic resonance imaging and positron emission tomography), clinical or behavioral tests (e.g., assessments of muscle weakness, tremor, muscle tone, motor skills, or memory), or laboratory tests.

Methods for measuring PGC-1α induction and activity are known in the art and are provided in the examples below. See, for example, Ruiz et al. (2012) A cardiac-specific robotized cellular assay identified families of human ligands as inducers of PGC-1α expression and mitochondrial biogenesis PLoS One: 7: e46753. doi: 10.1371/journal.pone.0046753. 3. PGC-1α levels can be assessed directly using for example an antibody to PGC-1α or other means of detection. PGC-1α activity can be detected including by way of example by assessing modulation of mitochondrial function, e.g., oxidative metabolism and can be assessed by detecting the activity or expression of a mitochondrial gene, e.g., LDH-2, ATP5j, or the like.

The term effective amount, as used throughout, is defined as any amount necessary or sufficient to produce a desired physiologic response. By way of example, the systemic dosage of the fenofibrate or analog thereof can be 1-1000mg daily, including for example, 300 to 400 mg daily (administered for example in 1-5 doses). One of skill in the art would adjust the dosage as described below based on specific characteristics of the inhibitor, the subject receiving it, the mode of administration, type and severity of the disease to be treated or prevented, and the like. Furthermore, the duration of treatment can be for days, weeks, months, years, or for the life span of the subject. For example, administration to a subject with or at risk of developing a neurodegenerative disease could be at least daily (e.g., once, twice, three times per day) for weeks, months, or years so long as the effect is sustained and side effects are manageable.

Effective amounts and schedules for administering fenofibrate or analogs thereof can be determined empirically and making such determinations is within the skill in the art. The dosage ranges for administration are those large enough to produce the desired effect in which one or more symptoms of the disease or disorder are affected (e.g., reduced or delayed). The dosage should not be so large as to cause substantial adverse side effects, such as unwanted cross-reactions, cell death, and the like. Generally, the dosage will vary with the type of neurodegenerative disease, the species, age, body weight, general health, sex and diet of the subject, the mode and time of administration, rate of excretion, drug combination, and severity of the particular condition and can be determined by one of skill in the art. The dosage can be adjusted by the individual physician in the event of any contraindications. Dosages can vary, and can be administered in one or more dose administrations daily.

The disclosure also provides a pharmaceutical pack or kit comprising packaging and/or one or more containers filled with one or more of the ingredients of the pharmaceutical compositions. Optionally the pharmaceutical pack or kit includes a second therapeutic agent as described above (e.g., L-dopa). Instructions for use of the composition can also be included.

Provided herein is a pharmaceutical composition comprising an effective amount of fenofibrate or analog thereof in a pharmaceutically acceptable carrier. The term carrier means a compound, composition, substance, or structure that, when in combination with a compound or composition, aids or facilitates preparation, storage, administration, delivery, effectiveness, selectivity, or any other feature of the compound or composition for its intended use or purpose. For example, a carrier can be selected to minimize any degradation of the active ingredient and to minimize any adverse side effects in the subject. Such pharmaceutically acceptable carriers include sterile biocompatible pharmaceutical carriers, including, but not limited to, saline, buffered saline, artificial cerebral spinal fluid, dextrose, and water.

Depending on the intended mode of administration, the pharmaceutical composition can be in the form of solid, semi-solid, or liquid dosage forms, such as, for example, tablets, suppositories, pills, capsules, powders, liquids, aerosols, or suspensions, preferably in unit dosage form suitable for single administration of a precise dosage. The compositions will include a therapeutically effective amount of the compound described herein or derivatives thereof in combination with a pharmaceutically acceptable carrier and, in addition, can include other medicinal agents, pharmaceutical agents, carriers, or diluents. By pharmaceutically acceptable is meant a material that is not biologically or otherwise undesirable, which can be administered to an individual along with the selected compound without causing unacceptable biological effects or interacting in a deleterious manner with the other components of the pharmaceutical composition in which it is contained.

As used herein, the term carrier encompasses any excipient, diluent, filler, salt, buffer, stabilizer, solubilizer, lipid, or other material well known in the art for use in pharmaceutical formulations. The choice of a carrier for use in a composition will depend upon the intended route of administration for the composition. The preparation of pharmaceutically acceptable carriers and formulations containing these materials is described in, e.g., Remington's Pharmaceutical Sciences, 21st Edition, ed. University of the Sciences in Philadelphia, Lippincott, Williams & Wilkins, Philadelphia Pa., 2005. Examples of physiologically acceptable carriers include buffers such as phosphate buffers, citrate buffer, and buffers with other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN® (ICI, Inc.; Bridgewater, New Jersey), polyethylene glycol (PEG), and PLURONICSTM (BASF; Florham Park, NJ).

Compositions containing the compound described herein or pharmaceutically acceptable salts or prodrugs thereof suitable for parenteral injection can comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions can also contain adjuvants such as preserving, wetting, emulsifying, and dispensing agents. Prevention of the action of microorganisms can be promoted by various antibacterial and antifungal agents, for example, parabens, chlorobutanol, phenol, sorbic acid, and the like. Isotonic agents, for example, sugars, sodium chloride, and the like can also be included. Prolonged absorption of the injectable pharmaceutical form can be brought about by the use of agents delaying absorption, for example, aluminum monostearate and gelatin.

Solid dosage forms for oral administration of the compounds described herein or pharmaceutically acceptable salts or prodrugs thereof include capsules, tablets, pills, powders, and granules. In such solid dosage forms, the compounds described herein or derivatives thereof is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alignates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example, paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms can also comprise buffering agents.

Solid compositions of a similar type can also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others known in the art. They can contain opacifying agents and can also be of such composition that they release the active compound or compounds in a certain part of the intestinal tract in a delayed manner. Examples of embedding compositions that can be used are polymeric substances and waxes. The active compounds can also be in micro-encapsulated form, if appropriate, with one or more of the above-mentioned excipients.

Liquid dosage forms for oral administration of the compounds described herein or pharmaceutically acceptable salts or prodrugs thereof include pharmaceutically acceptable emulsions, solutions, suspensions, syrups, and elixirs. In addition to the active compounds, the liquid dosage forms can contain inert diluents commonly used in the art, such as water or other solvents, solubilizing agents, and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, sesame oil, glycerol, tetrahydrofurfuryl alcohol, polyethyleneglycols, and fatty acid esters of sorbitan, or mixtures of these substances, and the like.

Besides such inert diluents, the composition can also include additional agents, such as wetting, emulsifying, suspending, sweetening, flavoring, or perfuming agents.

Suspensions, in addition to the active compounds, can contain additional agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions of the compounds described herein or pharmaceutically acceptable salts or prodrugs thereof for rectal administrations are optionally suppositories, which can be prepared by mixing the compounds with suitable non-irritating excipients or carriers such as cocoa butter, polyethyleneglycol or a suppository wax, which are solid at ordinary temperatures but liquid at body temperature and therefore, melt in the rectum or vaginal cavity and release the active component.

Also provided herein is a method of screening for an agent that promotes neuroprotection comprising contacting a cell with an agent to be screened and detecting PGC-1α level or activity in the cell, an increase in PGC-1α level or activity indicating the agent promotes neuroprotection. Optionally the cell is a neuron, glial cell or mononuclear blood cell.

As used throughout by control is meant a value from a subject lacking the neurodegenerative disease or a known control value exemplary of a population of subjects lacking the neurodegenerative disease. In some cases as described above, a control value can be from the same subject before the onset of a neurodegenerative disease or before the beginning of therapy therefor.

Throughout, treat, treating, and treatment refer to a method of reducing or delaying one or more effects or symptoms of a neurodegenerative disease. The subject can be diagnosed with the disease. Treatment can also refer to a method of reducing the underlying pathology rather than just the symptoms. The effect of the administration to the subject can have the effect of but is not limited to reducing one or more symptoms of the neurodegenerative disease or disorder, a reduction in the severity of the neurological disease or injury, the complete ablation of the neurological disease or injury, or a delay in the onset or worsening of one or more symptoms. For example, a disclosed method is considered to be a treatment if there is about a 10% reduction in one or more symptoms of the disease in a subject when compared to the subject prior to treatment or when compared to a control subject or control value. Thus, the reduction can be about a 10, 20, 30, 40, 50, 60, 70, 80, 90, 100%, or any amount of reduction in between.

As used throughout, by subject is meant an individual. Preferably, the subject is a mammal such as a primate, and, more preferably, a human. Non-human primates are subjects as well. The term subject includes domesticated animals, such as cats, dogs, etc., livestock (for example, cattle, horses, pigs, sheep, goats, etc.) and laboratory animals (for example, ferret, chinchilla, mouse, rabbit, rat, gerbil, guinea pig, etc.). Thus, veterinary uses and medical formulations are contemplated herein.

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including in the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

### Examples

### Example 1

Fenofibrate, a drug approved by the FDA to reduce blood cholesterol levels, was used as a molecule that can induce PGC-1α gene expression. In the dopaminergic neuronal cell line MN9D, fenofibrate increased PGC-1α in a dose-dependent manner (Figure 1A, upper left panel) and promoted a small increase in mitochondrial content (Figure 1B). In addition, fenofibrate robustly protected MN9D cells from 6-hydroxydopamine (6-OHDA)-induced oxidative stress mediated cell death (Figure 1C). Similarly, fenofibrate induced PGC-1α gene expression in the microglial cell line BV2 also in a dose-dependent manner (Figure 2A) and significantly inhibited LPS-induced IL-1β up-regulation (Figure 2B). In addition, using siRNA knockdown of PGC-1α, fenofibrate-mediated anti-inflammatory effect in BV2 cells was shown to require PGC-1α, as shown by the reduction in PGC-1α gene expression (Figure 3A) and I1-1β gene expression (Figure 3B). The PPARα antagonist GW6471 *fails* to suppress fenofibrate mediated PGC-1α upregulation and anti-inflammatory effects in BV2 cells (Figure 4), suggesting that the fenofibrate effect in BV2 cells is PPARα independent.

### Reagents

Fenofibrate was purchased from Sigma (Cat# F6020). PPARα antagonist was purchased from Sigma (Cat# G5045). IL-1β (Mm00434228-ml) and PGC-1α RT-PCR assays were purchased from Life Technologies (Carlsbad, CA). Cell culture reagents were also obtained from Life Technologies.

### Cell culture

A mouse midbrain cell line, MN9D, was used to evaluate neuroprotection effect of fenofibrate. MN9D cells were cultured in Dulbecco's Modified Eagle Medium (DMEM) supplemented with 10 % fetal bovine serum, and 3.7 g/L sodium bicarbonate at 37 °C, 5 % carbon dioxide. BV-2 cells (murine microglia) were maintained in DMEM supplemented with 10 % fetal bovine serum and antibiotics (penicillin, 100U/mL, streptomycin 100µg/mL). Antibiotics were omitted for the fenofibrate evaluation study.

### Example 2

Fenofibrate crosses the blood-brain barrier and exerts neuroprotective effects on the midbrain dopaminergic neurons that are affected in Parkinson's Disease (PD). Fenofibrate-mediated PGC-1α upregulation is posited to be a safe and effective intervention for neurodegenerative diseases, like PD, associated with PGC-1α deficiency and mitochondrial dysfunction.

The neuroprotective effects of fenofibrate in mediating PGC-1α up-regulation are evaluated in an MPTP toxicant mouse model of PD. MPTP is a potent neurotoxicant extensively used to model PD. MPTP inhibits mitochondrial Complex I, inducing ROS and leading to cell death. There are mainly three types of MPTP intoxication protocols: acute, subacute and chronic. Strong evidence suggests that 14-day chronic MPTP i.p. infusion protocol reproduces more accurately the pathological characteristics of early stage PD. Thus preclinical evaluation of a small molecule PGC-1α activator in a chronic MPTP toxicant model is key to the development of a mitochondrial strategy for early intervention in PD. Transgenic mice are used which are wild type or hemizygous (PGC-1α+/-) for the PGC-1α gene and which also harbor a transgene which reports Nrf1a,b/Nrf2 mediated transcriptional responses (antioxidant response element driven human placental alkaline phosphatase expression; AREhPLAP) (33). Nrf1/Nrf2 transcription lies downstream of PGC-1α and mediates the type II antioxidant response. Transgenic AREhPLAP mice with normal PGC-1α expression (C57BL6 background), or unique compound transgenic mice (AREhPLAP::PGC-1α+/-; C57BL6 background) with deficient PGC-1α expression are used for MPTP toxicant modeling. AREhPLAP mice allow easy monitoring of oxidative stress in affected brain regions by measurement of hPLAP. The use of AREhPLAP::PGC-1α+/- mice allows for the study of fenofibrate restoration of physiological levels of PGC-1α expression in the setting of half the genetic complement of endogenous PGC-1α. Prevention and protection are assessed in the chronic MPTP intoxicated mouse model of PD. To test preventive intervention, transgenic AREhPLAP or AREhPLAP::PGC-1α+/- mice (all C57BL/6) receive treatment of either oral fenofibrate at various doses (0, 1, 10 and 100 mg/kg) or control gavage for 28 days. Ten (10) days post the beginning of fenofibrate treatment, the mice are implanted i.p. with 14-day osmotic mini-pump (saline solution control: n=16; MPTP, 46mg/kg/daily: n=16). Motor coordination is tested in an accelerating Rota-Rod treadmill and mouse movement monitored by the AccuScan Digiscan System to detect horizontal and vertical movement 1 day before and 18 days post the MPTP infusion begins. One day later animals are sacrificed and brains are harvested for histopathologic evaluation. A subset of brains (n=8/group) are fixed and sectioned for immunochemistry analysis. To determine dopamine (DA) neuron number and terminals, brain sections containing SN and STR regions are stained for tyrosine hydroxylase (TH) followed by stereo logical enumeration of DA neurons in the SN and measurement of TH density in the STR with a computer-assisted image analysis system and stereological software. TUNEL staining is performed to determine apoptotic neuronal cell death. To characterize glial activation profiles, immunostaining for microglia (Iba1 IHC) and astrocytes (GFAP IHC) in the SN is used. Oxidative stress response is determined by hPLAP IHC. (II). The other subset of brains (n=8/group) are subject to STR and SN microdissection for biochemical and neurochemical assays of DA (and metabolites) content (HPLC measurement in STR and SN); PGC-1α expression (western blot); apoptotic protein levels (Western blot for cleaved caspase-3, PARP, Bcl-2, and Bax); stress response/survival genes (PKC, LRG-12, gadd45-β, TGF-β, NGF, BDNF, GDNF, and VIP); oxidative and anti-oxidative genes (SOD, Cat, NQO1, GST, Nrf2); and pro-inflammatory or anti-inflammatory genes (IL-1β, IL-6, TNF-α, NOS-II, COX-2, CCL2, Notch1, CCR2, arginase, Mmr, and IL10) expression (TaqMan@qRT-PCR arrays; 384-Well Micro Fluidic Cards). hPLAP is quantified by westerns and qRTPCR. Immunoreactivity signals from Western blotting are quantified by a Gel-Document Imaging System (BioRad).

For protective intervention, animals receive 14-day MPTP infusion (saline solution control: n=16; MPTP, 46mg/kg/daily: n=16) and then begin administration of oral drug treatment (0, 1, 10 and 100 mg/kg) for 28 days. The functional outcomes of treatments are evaluated by comparing Rotarod performance and locomotor activities 1 day before and 41 days post MPTP infusion begins. One day later animal are sacrificed and brains will be harvested for post-mortem neuropathology analysis including PGC-1α, phase II detoxification genes, mitochondrial function and pro/anti-inflammation gene profiling, PGC-1α and hPLAP protein expression, and enumeration of SN tyrosine hydroxylase (TH) positive neurons and STR TH density as described above.

### Rota-Rod Performance

Motor coordination is tested on an accelerating Rota-Rod treadmill (Columbus Instruments, Columbus, OH). Mice are tested at a constant speed of 5 rpm for 30 sec, and the speed will be then be accelerated by 0.1 revolutions per sec. The time at which each mouse falls off the rod is automatically recorded.

### Locomotor Activities

Mouse movements are monitored by the AccuScan Digiscan System (AccuScan Instruments, Inc., Columbus, OH) to detect horizontal and vertical movement. Data collected by computer include number of horizontal activity, number of vertical activity and total distance traveled.

### Immunohistochemistry

Serial frozen sections of the entire midbrain (30 µm) are cut from the rostral to the caudal end and subjected to immunostaining with TH, NeuN, GFAP, CD11 antibodies, followed by corresponding second antibody and standard ABC procedures. For unbiased cell counting, the dissector technique with Stereo-Investigator Operation System (MicroBrightfield Inc., Williston VT) is used to estimate the number of TH-positive neurons in SN.

### TaqMan microfluidics qRT-PCR

RNA is treated with RQ1 DNase (Promega, Madison, WI) to degrade any contaminating genomic DNA, followed by phenol:chloroform extraction and ethanol/LiCl precipitation. RNA integrity is determined by using Bioanalyzer technology (LCCC; Georgetown University Medical Center). One microgram of total RNA is reverse-transcribed in a 100-µl reaction with the Applied Biosystems High- Capacity cDNA Archive Kit. The quality of cDNA will be checked by PCR of β-actin. A 10-µl aliquot cDNA from each sample will be added to 90 µl Taqman Universal PCR master mix and loaded onto a Taqman Low Density Array Micro-Fluidic Card preloaded with probes and primers for target genes (PKC, LRG-12, gadd45-β, TGF-β, NGF, BDNF, GDNF and VIP, IL-1β, IL-6, TNF-α, NOS-II, COX-2, CCL2, Notch1, CCR2, arginase, Mmr, and Hes1) and one endogenous control (18sRNA). The real-time PCR reaction is run on the ABI Prism 7900HT Sequence Detection System (Applied Biosystems, Foster City, CA). The results are analyzed with the relative quantification ΔΔCT method, normalizing samples to treatment controls.

### Biochemical Assays

(1) STR DA levels determination are measured by HPLC. (2) Cell death detection kits are used for TUNEL assay. (3) Phosphorylation of RET and AKT, apoptotic protein levels (cleaved caspase-3, PARP, Bcl-2 and Bax in SN tissues) are determined by Western blot. (4) BDNF, GDNF, and VIP are measured by ELISA.

### Example 3

Fenofibrate induces PGC-1α gene expression a microglial cell line BV2 and inhibits LPS-mediated pro-inflammatory cytokine IL-1β production in a dose-dependent manner. In addition, with siRNA knockdown of PGC-1α, the fenofibrate-mediated anti-inflammatory effect in BV2 cells is shown to require PGC-1α. Considering that siRNA only partially knocks down PGC-1α gene expression, the use of primary CNS cells derived from homozygous and heterozygous PGC-1α knockout mice (PGC-1α-/- and PGC-1α-/+) would provide a more definitive proof of the indispensable role of PGC-1α in mediating fenofibrate effect. Heterozygous PGC-1α knockout mice (PGC-1α-/+) from the Jackson Laboratory (B6.129-Ppargc1atm1Brsp/J) (Bar Harbor, ME) were bred (PGC-1α-/+ x PGC-1α-/+) and primary microglial cells from heterozygous and wild type postnatal mice were isolated and cultured. The cells were treated with fenofibrate at various concentrations overnight followed by 0.1 ng/mL LPS for 1 hour. Total RNA was isolated and proinflammatory cytokine IL-1β and TNFα gene expression was determined by RT-PCR. The results showed that fenofibrate exerted similar anti-inflammation protection effects in both WT (PGC-1α+/+) and heterozygous (PGC-1α+/-) primary microglia (Figure 5A, 5B, 5D and 5E).

### Reagents

Tissue culture material including media, phosphate-buffered saline (PBS), and fetal bovine serum (FBS) were obtained from Life Technologies. Lipopolysaccharides (LPS) from Escherichia coli LPS was purchased from Sigma (Cat# L6529). Antibodies were purchased from Santa Cruz Biotechnology.

### Cell culture

Cerebral cortices of neonatal mice (1-day old; PGC-1α+/+ or PGC-1α+/-) were stripped of meninges and minced in Hepes balanced salt solution (HBSS; Mediatech Inc., Herndon, VA). Cells were dissociated in minimum essential media (MEM; Invitrogen, Frederick, MD) containing Earle's salts, 1-glutamine, 0.01% pyruvate, 0.6% glucose, 4% fetal bovine serum, and 6% horse serum (complete medium), centrifuged, resuspended, and plated into flasks containing 10 ml complete medium at a density of one brain per T75 flask. Cultures were grown at 37°C under 5% CO₂. After 1 day, the flasks were tapped gently to remove cell debris, media removed and replaced with fresh complete media. Cultures were grown as above for approximately 12 days at which time the microglia were harvested by tapping the flasks and collecting the microglia-enriched containing medium. Microglia were pelleted by centrifugation (1,000 rpm, 5 min), resuspended in MEM containing 0.01% pyruvate, 0.6% glucose, and 5% fetal bovine serum and enumerated.

BV-2 cells (murine microglia) were maintained in Dulbecco's Modified Eagle Medium supplemented with 10 % fetal bovine serum and antibiotics (penicillin, 100U/mL, streptomycin 100µg/mL). Antibiotics were omitted for the fenofibrate evaluation study.

### Example 4

AMPK is an energy sensor and can phosphorylate PGC-1α. It is plausible that AMPK phosphorylates PGC-1α and that PGC-1α then acts through positive feedback, binding to myocyte enhancer factor (MEF)-binding site to increase its own gene expression. Thus to determine whether AMPK signaling pathway was involved in mediating fenofibrate effect in CNS cells, BV2 cells were treated with various doses of fenofibrate for 1 hour and then cell lysates were collected for Western blotting analysis. AMPK phosphorylation was increased in response to fenofibrate treatment in a dose-dependent manner (Figure 2A). Next, BV2 cells were treated with AMPK inhibitor compound C for 0.5 hour followed by fenofibrate treatment for another 1 hour. A concentration-dependent inhibitory effect of compound C on AMPK phosphorylation was observed (Figure 2B). The inhibitory effect of compound C on fenofibrate mediated anti-inflammation in BV2 cells was tested. Compound C was added to BV2 cell cultures for 0.5 hour followed by fenofibrate treatment overnight. Compound C weakened the anti-inflammatory effects of fenofibrate in BV2 cells in a dose dependent manner (Figure 2C). These data suggest that AMPK activation plays a role in mediated fenofibrate effects in CNS cells.

## Claims

1. Fenofibrate for use in the treatment of a neurodegenerative disease by inducing PGC-1α expression in a neural cell or population of neural cells of a subject having a neurodegenerative disease and decreased PGC-1α expression, as compared to a subject not having the neurodegenerative disease.

2. Use of fenofibrate for inducing PGC-1α expression in vitro in a neural cell or population of neural cells.

3. The fenofibrate for the use of claim 1, or the use of claim 2, wherein the induction of PGC-1α is PPARα independent.

4. The fenofibrate for the use of claim 1, or the use of claim 2, wherein the neural cell or population of neural cells comprises one or more neurons, and optionally, wherein the neuron or neurons are dopaminergic neurons.

5. The fenofibrate for the use of claim 1, or the use of claim 2, wherein the neural cell or population of neural cells comprises one or more glial cells.

6. The fenofibrate for the use of claim 1, or the use of claim 2, wherein the fenofibrate
reduces one or more effects of oxidative stress;
increases a level of phosphorylated AMPK;
increases the number of mitochondria;
increases neural cell viability; or
provides an anti-inflammatory effect.

7. A composition for use in the treatment of a neurodegenerative disease in a subject, wherein the composition comprises fenofibrate in an amount sufficient to induce PGC-1α expression in a neural cell or population of neural cells of the subject, and the subject has a neurodegenerative disease and decreased PGC-1α expression, as compared to a subject who does not have the neurodegenerative disease.

8. The composition for the use of claim 7, wherein the subject has an early stage neurodegenerative disease.

9. The composition for the use of any one of claims 7 or 8, wherein the neurodegenerative disease is selected from the group consisting of Parkinson's Disease, Parkinson-plus syndrome, familial dementia, Alzheimer's Disease, Huntington's Disease, multiple sclerosis, dementia with Lewy bodies, Mild Cognitive Impairment, retinal neurodegeneration, and Amyotrophic Lateral Sclerosis.

10. The composition for the use of claim 9, wherein the Parkinson-plus syndrome is selected from the group consisting of multiple system atrophy (MSA), progressive supranuclear palsy (PSP), and corticobasal degeneration (CBD).

11. The composition for the use of any one of claims 7-10, wherein the fenofibrate is administered systemically, and optionally, administered orally.

12. The composition of any one of claims 7-11, wherein the effective amount of the fenofibrate induces PGC-1α expression in neural cells.

13. The composition for use of any one of claims 7-12, wherein the fenofibrate is administered daily.

14. The composition for use of any one of claims 7-13, further comprising administering a second therapeutic agent to the subject, wherein the second therapeutic agent is selected from the group consisting of levadopa, a dopamine agonist, an anticholinergic agent, a monoamine oxidase inhibitor, a COMT inhibitor, amantadine, rivastigmine, an NMDA antagonist, a cholinesterase inhibitor, riluzole, an anti-psychotic agent, an antidepressant, and tetrabenazine.

## Patentansprüche

1. Fenofibrat zur Verwendung bei der Behandlung einer neurodegenerativen Krankheit durch Induktion der PGC-1α-Expression in einer Nervenzelle oder einer Population von Nervenzellen eines Subjekts mit einer neurodegenerativen Krankheit und verminderter PGC-1α-Expression im Vergleich zu einem Subjekt ohne die neurodegenerative Krankheit.

2. Verwendung von Fenofibrat zur Induktion der PGC-1α-Expression in vivo in einer Nervenzelle oder einer Population von Nervenzellen.

3. Fenofibrat zur Verwendung nach Anspruch 1 oder Verwendung nach Anspruch 2, wobei die Induktion von PGC-1α PPARα-unabhängig ist.

4. Fenofibrat zur Verwendung nach Anspruch 1 oder Verwendung nach Anspruch 2, wobei die Nervenzelle oder die Population von Nervenzellen ein oder mehrere Neuronen umfasst und wobei das Neuron bzw. die Neuronen dopaminerge Neuronen sind.

5. Fenofibrat zur Verwendung nach Anspruch 1 oder Verwendung nach Anspruch 2, wobei die Nervenzelle oder die Population von Nervenzellen eine oder mehrere Gliazellen umfasst.

6. Fenofibrat zur Verwendung nach Anspruch 1 oder Verwendung nach Anspruch 2, wobei das Fenofibrat eine oder mehrere Auswirkungen von oxidativem Stress reduziert,
die Konzentration an phosphorylierter AMPK erhöht,
die Anzahl an Mitochondrien erhöht,
die Lebensfähigkeit von Nervenzellen erhöht oder
eine entzündungshemmende Wirkung bereitstellt.

7. Zusammensetzung zur Verwendung bei der Behandlung einer neurodegenerativen Krankheit in einem Subjekt, wobei die Zusammensetzung Fenofibrat in einer Menge umfasst, die für die Induktion der PGC-1α-Expression in einer Nervenzelle oder einer Population von Nervenzellen des Subjekts ausreicht und das Subjekt eine neurodegenerative Krankheit und verminderte PGC-1α-Expression im Vergleich zu einem Subjekt ohne die neurodegenerative Krankheit hat.

8. Zusammensetzung zur Verwendung nach Anspruch 7, wobei das Subjekt eine neurodegenerative Krankheit im Frühstadium hat.

9. Zusammensetzung zur Verwendung nach Anspruch 7 oder 8, wobei die neurodegenerative Krankheit aus der aus Parkinson-Krankheit, Parkinson-plus-Syndrom, familiärer Demenz, Alzheimer-Krankheit, Chorea Huntington, multipler Sklerose, Demenz mit Lewy-Körperchen, milder kognitiver Störung, retinaler Neurodegeneration und amyotropher Lateralsklerose bestehenden Gruppe ausgewählt ist.

10. Zusammensetzung zur Verwendung nach Anspruch 9, wobei das Parkinson-plus-Syndrom aus der aus multipler Systematrophie (MSA), progressiver Blickparese (Progressive Supranuclear Palsy, PSP) und kortikobasaler Degeneration (Corticobasal Degeneration, CBD) bestehenden Gruppe ausgewählt ist.

11. Zusammensetzung zur Verwendung nach einem der Ansprüche 7-10, wobei das Fenofibrat systemisch verabreicht wird und gegebenenfalls oral verabreicht wird.

12. Zusammensetzung nach einem der Ansprüche 7-11, wobei die wirksame Menge des Fenofibrats die PGC-1α-Expression in Nervenzellen induziert.

13. Zusammensetzung zur Verwendung nach einem der Ansprüche 7-12, wobei das Fenofibrat täglich verabreicht wird.

14. Zusammensetzung zur Verwendung nach einem der Ansprüche 7-13, weiterhin umfassend die Verabreichung eines zweiten Therapeutikums an das Subjekt, wobei das zweite Therapeutikum aus der aus Levadopa, einem Dopaminagonisten, einem Anticholinergikum, einem Monoaminoxidasehemmer, einem COMT-Inhibitor, Adamantin, Rivastigmin, einem NMDA-Antagonisten, einem Cholinesterasehemmer, Riluzol, einem Antipsychotikum, einem Antidepressivum und Tetrabenazin bestehenden Gruppe ausgewählt ist.

## Revendications

1. Fénofibrate pour utilisation dans le traitement d'une maladie neurodégénérative par induction de l'expression de PGC-1α dans une cellule neuronale ou une population de cellules neuronales d'un sujet ayant une maladie neurodégénérative et une expression de PGC-1α réduite, par rapport à un sujet n'ayant pas la maladie neurodégénérative.

2. Utilisation de fénofibrate pour l'induction de l'expression de PGC-1α *in vitro* dans une cellule neuronale ou une population de cellules neuronales.

3. Fénofibrate pour l'utilisation selon la revendication 1, ou l'utilisation selon la revendication 2, où l'induction de PGC-1α est indépendante de PPARα.

4. Fénofibrate pour l'utilisation selon la revendication 1, ou l'utilisation selon la revendication 2, où la cellule neuronale ou la population de cellules neuronales comprend un ou plusieurs neurones, et facultativement, où le neurone ou les neurones sont des neurones dopaminergiques.

5. Fénofibrate pour l'utilisation selon la revendication 1, ou l'utilisation selon la revendication 2, où la cellule neuronale ou la population de cellules neuronales comprend une ou plusieurs cellules gliales.

6. Fénofibrate pour l'utilisation selon la revendication 1, ou l'utilisation selon la revendication 2, où le fénofibrate réduit un ou plusieurs effets du stress oxydatif ; augmente un taux d'AMPK phosphorylé ; augmente le nombre de mitochondries ; augmente la viabilité de cellules neuronales ; ou produit un effet anti-inflammatoire.

7. Composition pour utilisation dans le traitement d'une maladie neurodégénérative chez un sujet, où la composition comprend du fénofibrate en une quantité suffisante pour induire l'expression de PGC-1α dans une cellule neuronale ou une population de cellules neuronales du sujet, et le sujet a une maladie neurodégénérative et une expression de PGC-1α réduite, par rapport à un sujet qui n'a pas la maladie neurodégénérative.

8. Composition pour l'utilisation selon la revendication 7, où le sujet a une maladie neurodégénérative à un stade précoce.

9. Composition pour l'utilisation selon l'une quelconque des revendications 7 ou 8, où la maladie neurodégénérative est choisie dans le groupe constitué de la maladie de Parkinson, le syndrome Parkinson plus, la démence familiale, la maladie d'Alzheimer, la maladie de Huntington, la sclérose en plaques, la démence avec corps de Lewy, un trouble cognitif léger, la neurodégénérescence rétinienne, et la sclérose latérale amyotrophique.

10. Composition pour l'utilisation selon la revendication 9, où le syndrome Parkinson plus est choisi dans le groupe constitué de l'atrophie multisystémisée (AMS), la paralysie supranucléaire progressive (PSP) et la dégénérescence corticobasale (DCB).

11. Composition pour l'utilisation selon l'une quelconque des revendications 7 à 10, où le fénofibrate est administré de façon systémique et, facultativement, administré par voie orale.

12. Composition pour l'utilisation selon l'une quelconque des revendications 7 à 11, où la quantité efficace du fénofibrate induit l'expression de PGC-1α dans des cellules neuronales.

13. Composition pour l'utilisation selon l'une quelconque des revendications 7 à 12, où le fénofibrate est administré chaque jour.

14. Composition pour l'utilisation selon l'une quelconque des revendications 7 à 13, comprenant en outre l'administration d'un deuxième agent thérapeutique au sujet, le deuxième agent thérapeutique étant choisi dans le groupe constitué des lévodopa, agoniste de dopamine, agent anticholinergique, inhibiteur de monoamine oxydase, inhibiteur de COMT, amantadine, rivastigmine, antagoniste de NMDA, inhibiteur de cholinestérase, riluzole, agent antipsychotique, antidépresseur et tétrabénazine.
